# EUROPÄISCHE PATENTANMELDUNG

(11) **EP 4 434 481 A1**
(43) Veröffentlichungstag der Anmeldung: **25.09.2024**
(21) Anmeldenummer: 24165081.1
(22) Anmeldetag: 21.03.2024
(51) Int. Cl.: A61B 18/12, A61B 17/00, A61B 18/00

(54) **ANSCHLUSSANORDNUNG FÜR EIN CHIRURGISCHES INSTRUMENT ODER IN EINEM CHIRURGISCHEN INSTRUMENT**

(30) Priorität: 21.03.2023 DE 102023107109
(71) Anmelder: Karl Storz SE & Co. KG, 78532 Tuttlingen (DE)
(72) Erfinder: GRÜNER, Sven Axel, 78532 Tuttlingen (DE); DIETERLE, Lisa, 78532 Tuttlingen (DE)

(57) **Zusammenfassung**

Die vorliegende Erfindung betrifft eine Anschlussanordnung für ein chirurgisches Instrument oder in einem chirurgischen Instrument, mit einer Instrumentenbasis, die zur Ansteuerung und/oder Bedienung eines Hoch-Frequenz-Werkzeugs des chirurgischen Instruments ausgebildet ist, mit einem Anschlusskörper eines Hoch-Frequenz-Anschlusses, wobei der Anschlusskörper zur vorbestimmten Ausrichtung in einer Anschlussachse des Hoch-Frequenz-Anschlusses innerhalb der Instrumentenbasis ausgebildet ist, wobei die Instrumentenbasis eine Aufnahme für den Anschlusskörper ausbildet und die Aufnahme einen Ausrichtungsabschnitt aufweist, wobei der Anschlusskörper einen korrespondierenden Ausrichtungsabschnitt aufweist, und wobei der Anschlusskörper durch Einführen in die Aufnahme durch die Ausrichtungsabschnitte in einem vorbestimmten Drehwinkel auf der Anschlussachse selbstausrichtbar ist.

Des Weiteren betrifft die Erfindung ein chirurgisches Instrument mit einer Anschlussanordnung.

## Beschreibung

### GEBIET DER ERFINDUNG

Die vorliegende Erfindung betrifft eine Anschlussanordnung für ein chirurgisches Instrument oder in einem chirurgischen Instrument. Des Weiteren betrifft die Erfindung ein chirurgisches Instrument mit einer Anschlussanordnung.

### TECHNISCHER HINTERGRUND

Chirurgische Instrumente werden für unterschiedliche Anwendungen eingesetzt. Beispielsweise können diese als mikroinvasive medizinische Instrumente ausgebildet sein und eine Handhabungseinrichtung an einem proximalen Ende, einen langen und in der Regel dünnen Schaft, der sich von dem proximalen Ende zu einem distalen Ende des Instruments erstreckt, und ein Werkzeug oder eine andere Wirkeinrichtung zum Greifen, Quetschen, Koagulieren, Schneiden, Stanzen oder für andere Anwendungen an dem distalen Ende des Instruments, umfassen. In dem Schaft verlaufen eine oder mehrere Übertragungseinrichtungen zum Übertragen einer Kraft und/oder eines Drehmoments von der Handhabungseinrichtung am proximalen Ende zu der Wirkeinrichtung am distalen Ende. So ist bei mikroinvasiven medizinischen Instrumenten mit elektrochirurgischer Funktion, insbesondere bei bipolaren elektrochirurgischen mikroinvasiven medizinischen Instrumenten, die Übertragungseinrichtung oft auch an einer Übertragung elektrischer Leistung von dem proximalen Ende zu dem distalen Ende beteiligt.

Vorteilhafterweise sind die Instrumente in einzelne Komponenten zerlegbar, sodass unterschiedliche Werkzeuge, unterschiedliche Schäfte und sonstige Anschlusselemente kombiniert werden können. Vorteilhafterweise kann daher ein System mit unterschiedlichen und daher vielseitigen Einsatzmöglichkeiten bereitgestellt werden.

Hochwertige mikroinvasive medizinische Instrumente sind zudem in der Regel wiederverwendbar. Um eine Reinigung nach der Verwendung zu vereinfachen, einen Austausch einer defekten Komponente und/oder eine alternative Verwendung unterschiedlicher Komponenten zu ermöglichen, ist ein hochwertiges mikroinvasives medizinisches Instrument vorteilhafterweise zerlegbar.

Zum einen muss bei einem zerlegbaren medizinischen Instrument, bei dem die Übertragungseinrichtung auch an der Übertragung elektrischer Leistung beteiligt ist, insbesondere am proximalen Ende des Instruments ein mechanisch trennbarer und sicher wieder herstellbarer elektrischer Kontakt zu der Übertragungseinrichtung herstellbar sein.

Weiterhin sollen nur Komponenten miteinander kombinierbar sein, die auch funktionsfähig eingesetzt werden können. So soll sichergestellt werden, dass bei einer Mehrzahl an unterschiedlichen Systemen, wie beispielsweise einem monopolar, einem bipolar oder einem stromlos betriebenen chirurgischen Instrument, nur diejenigen Komponenten miteinander kombinierbar sind, die zu demselben System gehören. Eine Fehlanwendung, eine Beschädigung und Ähnliches können dadurch verhindert werden.

So zeigt die Druckschrift DE 10 2017 124 775 A1 ein mikroinvasives medizinisches Instrument mit einer Instrumentenbasis und einem in die Instrumentenbasis einsteckbaren Zubehörschafts mit einer Übertragungseinrichtung zur Übertragung von elektrischer Leistung sowie zur Übertragung einer Kraft und/oder eines Drehmoments von einer proximalen Position zu einer distalen Position. In der Instrumentenbasis ist eine Kontakteinrichtung angeordnet, die an der dem Zubehörschaft abgewandten Seite mit einem Steckkontakt in Verbindung steht.

Nachteilig ist bei derartigen Instrumenten der Steckkontakt, beispielsweise ein Hoch-Frequenz-Anschluss, in der Instrumentenbasis, d. h. in dem Handgriffelement, fest montiert.

Daher existieren auch entnehmbare Hoch-Frequenz-Anschlüsse, die als Steckerelement in die Instrumentenbasis eingesteckt werden können und eine Anschlussanordnung ausbilden. Hierbei ist jedoch eine Montage oftmals aufwendig, da eine exakte Ausrichtung nötig ist.

### ZUSAMMENFASSUNG DER ERFINDUNG

Vor diesem Hintergrund liegt der vorliegenden Erfindung die Aufgabe zugrunde, eine verbesserte Anschlussanordnung anzugeben.

Erfindungsgemäß wird diese Aufgabe durch eine Anschlussanordnung mit den Merkmalen des Patentanspruchs 1 und mit einem chirurgischen Instrument mit den Merkmalen des Patentanspruchs 17 gelöst.

Demgemäß ist vorgesehen:
Eine Anschlussanordnung für ein chirurgisches Instrument oder in einem chirurgischen Instrument, mit einer Instrumentenbasis, die zur Ansteuerung und/oder Bedienung eines Hoch-Frequenz-Werkzeugs des chirurgischen Instruments ausgebildet ist, mit einem Anschlusskörper eines Hoch-Frequenz-Anschlusses, wobei der Anschlusskörper zur vorbestimmten Ausrichtung in einer Anschlussachse des Hoch-Frequenz-Anschlusses innerhalb der Instrumentenbasis ausgebildet ist, wobei die Instrumentenbasis eine Aufnahme für den Anschlusskörper ausbildet und die Aufnahme einen Ausrichtungsabschnitt aufweist, wobei der Anschlusskörper einen korrespondierenden Ausrichtungsabschnitt aufweist, und wobei der Anschlusskörper durch Einführen in die Aufnahme durch die Ausrichtungsabschnitte in einem vorbestimmten Drehwinkel auf der Anschlussachse selbstausrichtbar ist.
Ein chirurgisches Instrument, umfassend eine derartige Anschlussanordnung, wobei das chirurgische Instrument bipolar oder monopolar betrieben ist.

Die der vorliegenden Erfindung zugrundeliegende Erkenntnis besteht darin, dass eine rotative Festlegung des Hoch-Frequenz-Anschlusses in der Aufnahme der Instrumentenbasis von Vorteil ist, wenn dieser eindeutig und ohne weiteres Zutun montiert werden soll.

Die der vorliegenden Erfindung zugrundeliegende Idee besteht darin, ein Einführen des Hoch-Frequenz-Anschlusses in der Aufnahme in einem vorbestimmten Drehwinkel auf einer Anschlussachse des Hoch-Frequenz-Anschlusses durch Ausrichtungsabschnitte, welche an der Aufnahme und an dem Hoch-Frequenz-Anschluss angeordnet sind, in selbstausrichtender Weise zu ermöglichen.

Mit anderen Worten kann eine rotative Festlegung eines im Wesentlichen runden Hoch-Frequenz-Anschlusses in Form eines Steckers in der Aufnahme, welche als eine Art Bohrung oder Sacklochbohrung ausgebildet ist, derart erfolgen, dass eine Montage eindeutig ohne weitere Ausrichtungsmaßnahmen und/oder ohne wiederholtes Probieren möglich ist.

Hierbei ist ein korrekt ausgerichtetes Einführen zur Montage nicht zwingend erforderlich, um einen Betrieb des chirurgischen Instrumentes zu gewährleisten, da die Ausrichtungsabschnitte für eine selbsttätige rotatorische Ausrichtung beim Einführen sorgen. Der Hoch-Frequenz-Anschluss muss daher nicht in der exakt passenden Position ausgerichtet in die Instrumentenbasis eingeführt werden. Vorteilhaft werden auf diese Weise komplexe Führungsgeometrien zwischen dem Hoch-Frequenz-Anschluss und der Instrumentenbasis oder komplexe Ausrichtungswerkzeuge zur Montage vermieden.

Ferner ist es auf diese Weise vorteilhaft möglich, eine Verdrehsicherung des Hoch-Frequenz-Anschlusses relativ zu der Aufnahme auszubilden. Dadurch kann auf ein verstiften, verkleben oder auf eine andere form- oder stoffschlüssige Verbindung zwischen dem Hoch-Frequenz-Anschluss und der Aufnahme verzichtet werden, wodurch Arbeitsschritte bzw. Montageschritte eingespart und Ausrichtungsfehler vermieden werden können.

Der Hoch-Frequenz-Anschluss kann unterschiedliche Ausgestaltungen aufweisen. Insbesondere ist dieser als ein Steckerelement ausgebildet und weist einen im Wesentlichen runden Querschnitt auf. Der Querschnitt kann konisch zulaufen, wodurch auch die Herstellbarkeit durch ein Spritzgussverfahren optimiert werden kann.

Als Instrumentenbasis kann ein Handgriffelement vorgesehen sein, an welchem die weiteren Bauteile des chirurgischen Instruments befestigt bzw. montiert werden können. Alternativ oder zusätzlich kann es sich für roboterchirurgische Anwendungen bei der Instrumentenbasis selbst um eine Roboterschnittstelle oder um eine mit einer Roboterkupplung montierbare Instrumentenbasis handeln.

Vorteilhafterweise handelt es sich bei dem chirurgischen Instrument um ein bipolar betriebenes Instrument. In einer weiteren Ausführung kann das chirurgische Instrument ein monopolar betriebenes Instrument sein.

Als Anschlusskörper des Hoch-Frequenz-Anschlusses kann ein Grundkörper des Hoch-Frequenz-Anschlusses verstanden werden, an welchem alle anderen Bauteile des Hoch-Frequenz-Anschlusses, insbesondere die Pole und Kontakte, angebracht bzw. vormontiert oder vormontierbar sind.

Als Ausrichtungsabschnitt kann eine Bauteilgeometrie verstanden werden, die ein Ausrichten auf der Anschlussachse und/oder ein rotatives Festlegen in einem Drehwinkel um die Anschlussachse der beiden Bauteile gegeneinander, d. h. des Hoch-Frequenz-Anschlusses und der Instrumentenbasis, ermöglicht.

Als ein vorbestimmter Drehwinkel auf der Anschlussachse ist eine Ausrichtung in einem im montierten Zustand gewünschten Rotationswinkel zu verstehen, in welchem der Hoch-Frequenz-Anschluss korrekt ausgerichtet ist, wenn dieser vollständig in die Aufnahme eingeführt bzw. eingeschoben ist. Durch die Ausrichtungsabschnitte ist eine Selbstausrichtung des Hoch-Frequenz-Anschlusses in diesen vorbestimmten Rotationswinkel beim Einführen möglich. Dadurch können insbesondere auch Beschädigungen vermieden werden, die an dem Hoch-Frequenz-Anschluss, beispielweise an den Kontakten und an der Instrumentenbasis, durch ein nicht korrekt ausgerichtetes Einschieben resultieren können.

Ist der Hoch-Frequenz-Anschluss in der Instrumentenbasis, d. h. beispielsweise in einem Handgriff, montiert, ist es insbesondere wichtig, dass eine Drehstellung des Hoch-Frequenz-Anschlusses mit einer Sollstellung übereinstimmt, damit innenliegende Kontakte und/oder Durchgänge reibungslos mit der Instrumentenbasis und einem darin einführbaren Instrumentenschaft zusammenspielen bzw. diese korrekt kontaktieren.

Vorteilhafte Ausgestaltungen und Weiterbildungen ergeben sich aus den weiteren Unteransprüchen sowie aus der Beschreibung unter Bezugnahme auf die Figuren der Zeichnung.

Gemäß einer vorteilhaften Ausführungsform können die Ausrichtungsabschnitte als ein Vorsprung und ein dazu korrespondierender Rücksprung ausgebildet sein. Vorteilhafterweise kann so ein Formschluss zwischen dem Hoch-Frequenz-Anschluss und der Instrumentenbasis ausgebildet werden, bei welchem eine bereits vorhandene Geometrie ausgenutzt werden kann. So kann als ein Ausrichtungsabschnitt beispielsweise eine bereits vorhandene Nut verwendet werden, welche in dem Hoch-Frequenz-Anschluss, insbesondere in dem Anschlusskörper, ausgeformt ist, um beispielsweise andere Bauelemente aufzunehmen. Weiterhin kann eine Nut verwendet werden, die beispielsweise herstellungsbedingt in dem Hoch-Frequenz-Anschluss ausgeformt ist. Des Weiteren kann eine Nut bereits in der Aufnahme der Instrumentenbasis vorgesehen sein, welche nun als Ausrichtungsabschnitt verwendet werden kann. Dasselbe gilt beispielsweise für eine Nase oder eine Aufwölbung, welche herstellungsbedingt oder aus anderen Gründen bereits an dem Anschlusskörper oder in der Aufnahme ausgeformt ist. Folglich werden vorteilhafterweise für eine Verdrehsicherung keine eigene Geometrie und keine zusätzlichen Bauelemente benötigt, sondern es werden die bereits vorhandenen Merkmale ausgenutzt. Die Nut kann hierbei als Rücksprung, die Nase bzw. Aufwölbung als Vorsprung interpretiert werden.

Gemäß einer Weiterbildung kann der Vorsprung von einer Innenoberfläche der Aufnahme hervorstehen. Dadurch kann der Vorsprung an einer gewünschten Position und/oder über eine gewisse Länge der Aufnahme verlaufen. Ist ein Rücksprung an dem Anschlusskörper korrespondierend zu dem Vorsprung ausgebildet, erfolgt vorteilhafterweise eine Verdrehsicherung des Hoch-Frequenz-Anschlusses relativ zu der Instrumentenbasis.

Gemäß einer Ausführungsform kann der Vorsprung entlang der Anschlussachse ausgerichtet sein und eine längliche Form aufweisen. Vorteilhaft ist auf diese Weise ein ausgerichtetes Einführen des Anschlusskörpers in die Aufnahme möglich, da bereits zu einem frühen Zeitraum ein Kontakt zwischen den beiden Ausrichtungsabschnitten stattfindet.

Gemäß einer vorteilhaften Ausführungsform kann der Vorsprung über zumindest die Hälfte einer Länge der Aufnahme verlaufen, wobei die Länge in Richtung der Anschlussachse definiert ist. Der Vorsprung kann dabei mit einer Einführöffnung der Aufnahme kontaktieren sein, sodass bereits bei Kontakt des Anschlusskörpers mit der Aufnahme die Ausrichtung in dem vorbestimmten Drehwinkel auf der Anschlussachse quasi selbst ausgerichtet erfolgt. So kann optisch wahrgenommen werden, in welchem Winkel der Hoch-Frequenz-Anschluss in die Aufnahme eingeschoben werden muss.

Gemäß einer bevorzugten Ausführungsform kann der Vorsprung beabstandet zu einer Einführöffnung der Aufnahme angeordnet sein, sodass der Vorsprung ebenso einen Anschlag ausbildet, der ein Einschieben des Anschlusskörpers in die Aufnahme auf der Anschlussachse begrenzt. Der Vorsprung kann an dem Ende, welches in Richtung der Einführöffnung ausgerichtet ist, abgerundet ausgebildet sein, sodass bei Kontakt mit dem Rücksprung in dem Anschlusskörper ein vereinfachtes "Einfädeln" erfolgen kann. Die beiden Ausrichtungsabschnitte können daher aneinander abgleiten.

Vorteilhafterweise kann der Vorsprung als Negativform des Rücksprungs ausgebildet sein. Dadurch kann eine Relativverschiebung zwischen dem Hoch-Frequenz-Anschluss und der Instrumentenbasis verhindert werden, da durch die Ausrichtungsabschnitte, d. h. insbesondere den Vorsprung und den Rücksprung, eine Art Formschluss hergestellt werden kann. Insbesondere kann ein Verdrehen aus dem vorbestimmten Drehwinkel in dem eingebauten Zustand verhindert werden.

Gemäß einer besonders bevorzugten Ausführungsform kann der Vorsprung zur Ausrichtung des Anschlusskörpers in der Aufnahme zu der Einführöffnung konisch zulaufende Seitenkanten aufweisen. Vorteilhaft kann dadurch eine Form ausgebildet werden, die über ein Spritzgussverfahren hergestellt werden kann und weiterhin Vorteile bei der Montage aufweist. Durch eine konische Ausführung, d. h. eine Verjüngung in Richtung der Einführöffnung, ergibt sich bei der Montage der Vorteile, dass sich die Bauteile, d. h. der Anschlusskörper und die Aufnahme, leicht "finden". Wird der Anschlusskörper dann weiter in die Aufnahme eingeschoben, kann durch den zunehmend breiter werdenden Vorsprung, insbesondere als eine Art breiter werdende Rippe, ein Ausrichten des Anschlusskörpers zu einer endgültigen Position erfolgen, in welcher der Anschlusskörper final spielfrei verbleiben soll.

Gemäß einer vorteilhaften Ausführungsform kann der Anschlusskörper konisch ausgebildet sein und sich in Richtung eines Steckverbinders verjüngen. Dadurch ergeben sich beispielsweise Vorteile bei der Herstellung durch ein Spritzgussverfahren. Der Anschlusskörper kann beispielsweise aus einem isolierenden Kunststoff hergestellt sein. An dem Anschlusskörper, als eine Art Grundkörper, können zumindest zwei elektrische Pole angeordnet sein. Einer der Pole kann als ein Kontaktblech ausgebildet sein, das von einem Steckverbinder, an einem Ende des Anschlusskörpers, zu einem gegenüberliegenden Ende des Anschlusskörpers verläuft. An dem gegenüberliegenden Ende können Kontaktlaschen, insbesondere vier Kontaktlaschen, ausgebildet werden. Das Kontaktblech kann beispielsweise entlang eines Ausrichtungsabschnitts verlaufen. Dabei kann eine Ausformung im Anschlusskörper, welche zur Aufnahme des Kontaktblechs ausgebildet ist, ebenso als Ausrichtungsabschnitt dienen. Folglich kann eine bereits vorhandene Geometrie des Anschlusskörpers als Ausrichtungsabschnitt genutzt werden. Ein zweiter Pol kann ebenso von einem Ende zu einem gegenüberliegenden Ende am Anschlusskörper geführt werden, und läuft insbesondere innerhalb des Anschlusskörpers.

Gemäß einer Weiterbildung kann der Anschlusskörper zumindest eine schräge oder gekrümmte Einführkontur aufweisen, um ein Einführen der Ausrichtungsabschnitte zu ermöglichen, wenn der Anschlusskörper in einem Winkel ungleich des vorbestimmten Drehwinkels auf der Anschlussachse ausgerichtet ist.

Die Einführkontur ist insbesondere an dem gegenüberliegenden Ende am Anschlusskörper angeordnet, die zuerst in die Aufnahme eingeschoben wird. Folglich an dem Ende, an welchem nicht der Steckverbinder vorgesehen ist, sondern die Kontaktlaschen. Die Einführkontur formt insbesondere einen gekrümmten Randbereich des Anschlusskörpers auf, der geneigt zu der Anschlussachse ausgeformt ist, und insbesondere die Kontaktlaschen vor einer Beschädigung oder einem Verbiegen schützt. Die Einführkontur ist daher bevorzugt nicht entlang eines Querschnitts durch den Anschlusskörper ausgebildet, der quer, insbesondere rechtwinklig, zu der Anschlussachse verläuft. Vielmehr ist die Einführungskontur als eine schräge Angriffsfläche ausgeformt, die schräg zu einer rechtwinklig angeordneten Querschnittsfläche des Anschlusskörpers ausgerichtet ist.

Gemäß einer Ausführungsform kann der Rücksprung eine Aufnahme für ein Kontaktblech eines ersten Pols ausbilden, wobei das Kontaktblech eine geringere Dicke als eine Tiefe des Rücksprungs aufweist. Dadurch kann beispielsweise sichergestellt werden, dass eine ausreichend große Nut verbleibt, die als Ausrichtungsabschnitt dienen kann. Insbesondere kann der Rücksprung über die komplette Länge des Anschlusskörpers ausgebildet sein, während der Vorsprung eine geringere Länge aufweist.

Gemäß einer Ausführungsform kann in einem an die Einführkontur anschließenden Endbereich der Rücksprung stetig in die Einführkontur übergehen. Vorteilhaft ist auf diese Weise ein Kontaktieren des Anschlusskörpers mit der Aufnahme derart möglich, dass bei weiterem Einschieben des Anschlusskörpers in die Aufnahme zu einem frühen Zeitpunkt ein Ausrichten in dem vorbestimmten Drehwinkel erfolgen kann. Kontaktiert der Ausrichtungsabschnitt des Anschlusskörpers die Einführkontur, so wird der Ausrichtungsabschnitt des Anschlusskörpers in Richtung des Ausrichtungsabschnitts der Aufnahme geführt, wodurch ein korrektes Einführen bzw. "Einfädeln" der beiden Ausrichtungsabschnitte miteinander erfolgen kann.

Bei einer vorteilhaften Ausführungsform kann der Anschlusskörper Ausnehmungen aufweisen, die als Griff und/oder Kennzeichnung zur rotatorischen Ausrichtung in dem vorbestimmten Drehwinkel des Anschlusskörpers relativ zu der Aufnahme dienen. Beispielsweise kann der Anschlusskörper an den Ausnehmungen von einer Bedienperson in der Hand gehalten werden. Greift diese Person die Ausnehmungen, erfolgt "automatisch" eine korrekte Ausrichtung in dem vorbestimmten Drehwinkel, da beispielsweise die Ausnehmungen symmetrisch zu einer Längsachse des chirurgischen Instruments, und daher auch zu einer Längsachse der Instrumentenbasis, ausgerichtet sind. Die Ausnehmungen sind bevorzugt in dem Bereich angeordnet, der in Richtung eines Steckverbinders an dem Anschlusskörper verläuft. Insbesondere sind die Ausnehmungen daher nicht mit der Einführungskontur in Kontakt, und bevorzugt beanstandet zu der Einführungskontur ausgebildet. Dadurch ist die Einführung des Anschlusskörpers in die Aufnahme unbeeinflusst von den Ausnehmungen möglich.

Gemäß einer vorteilhaften Ausführungsform kann der Anschlusskörper zumindest teilweise zylindrisch ausgebildet sein. Beispielsweise kann es sich um eine kreiszylindrische Ausbildung handeln. Dadurch kann eine Art Steckerelement ausgebildet werden, das einen im Wesentlichen runden Querschnitt aufweist. Dabei ist es vorteilhaft, wenn der Anschlusskörper zumindest im vorderen Bereich, d. h. in dem Bereich, der zuerst in die Aufnahme geführt wird, zumindest teilweise zylindrisch ausgeführt ist. In einer weiteren Ausführungsform kann sich der Anschlusskörper in Richtung des Steckverbinders verjüngen.

Gemäß einer Weiterbildung können die Ausnehmungen einen Teil der Verjüngung ausbilden und an einer gegenüberliegenden Seite der Anschlusskörper eine zylindrische Oberfläche aufweisen. Dadurch kann die Einführungskontur unbeeinflusst von der Verjüngung ausgebildet sein, und gleichzeitig eine ergonomische Handhabung des Hoch-Frequenz-Anschlusses in Form eines Steckers ermöglicht werden.

Gemäß einer vorteilhaften Ausführungsform kann die Einführkontur die zylindrische Oberfläche schneiden. Während die zylindrische Oberfläche bevorzugt Rotation symmetrisch um die Anschlussachse verläuft, kann die Einführungskontur derart schräg auf der Anschlussachse ausgerichtet sein, dass diese nicht in einem Querschnitt rechtwinklig zu der Anschlussseite läuft. Dadurch wird ein Teil der zylindrischen Oberfläche entfernt, der beispielsweise Einführschrägen ausbilden kann, und einen Vorsprung in der Aufnahme in den Rücksprung in dem Anschlusskörper zu führen.

Gemäß einer vorteilhaften Ausführungsform kann der Anschlusskörper als einachsig entformbares Spritzgussteil ausgebildet sein. Da ist es vorteilhaft, werde alle Oberflächen in Richtung einer Achse konisch zusammenlaufen, sodass eine einfache Entformung möglich ist. Vorteilhafterweise sind ebenso keine scharfen Kanten an dem Anschlusskörper vorhanden, wodurch lediglich runde Oberflächen entstehen. Auch dadurch ergeben sich Vorteile beim Entformen nach dem Spritzgussverfahren.

Gemäß einer Weiterbildung kann die Instrumentenbasis als eine Verbindungsvorrichtung ausgebildet sein, beispielsweise zu einem als Handgriff ausgeführten manuellen Führungselement, zu einer Manipulatorkupplung oder zu einer Roboteraufnahme.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

### INHALTSANGABE DER ZEICHNUNG

Die vorliegende Erfindung wird nachfolgend anhand der in den schematischen Figuren der Zeichnung angegebenen Ausführungsbeispiele näher erläutert. Es zeigen dabei:
- Fig. 1: einen Längsschnitt durch ein chirurgisches Instrument;
- Fig. 2: eine Instrumentenbasis;
- Fig. 3: eine Aufnahme in einer Instrumentenbasis;
- Fig. 4: einen Querschnitt durch die Aufnahme aus Fig. 3;
- Fig. 5: einen Längsschnitt durch die Aufnahme aus Fig 3;
- Fig. 6: einen Anschlusskörper;
- Fig. 7: ein Längsschnitt durch den Anschlusskörper aus Fig. 6;
- Fig. 8: eine weitere Ansicht eines Anschlusskörpers;
- Fig. 9: eine weitere Ansicht eines Anschlusskörpers;
- Fig. 10: eine weitere Ansicht eines Anschlusskörpers;
- Fig. 11: eine weitere Ansicht eines Anschlusskörpers mit Steckverbinder;
- Fig. 12: eine weitere Ansicht eines Anschlusskörpers aus Fig. 11;
- Fig. 13: eine weitere Ansicht eines Anschlusskörpers; und
- Fig. 14: eine weitere Ansicht des Anschlusskörpers aus Fig. 13.

Die beiliegenden Figuren der Zeichnung sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts Anderes ausgeführt ist - jeweils mit denselben Bezugszeichen versehen.

### BESCHREIBUNG VON AUSFÜHRUNGSBEISPIELEN

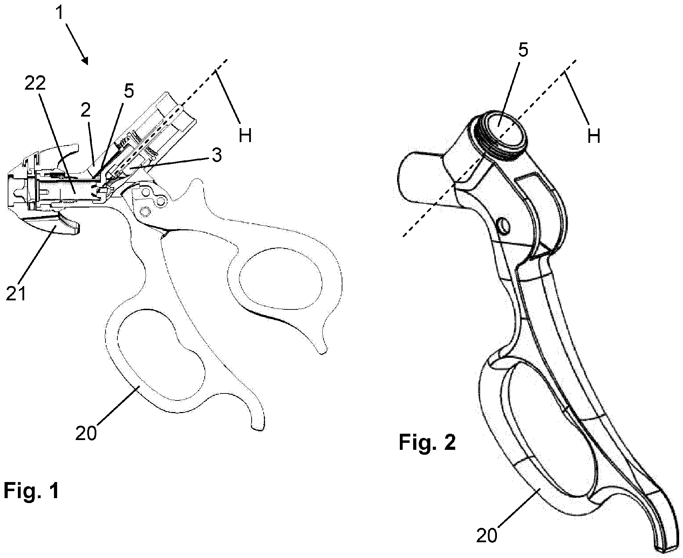

Fig. 1 zeigt einen Längsschnitt durch ein chirurgisches Instrument. Vorteilhafterweise handelt es sich bei dem chirurgischen Instrument um ein bipolar betriebenes Instrument. Eine Ausführung als monopolar betriebenes Instrument ist ebenso denkbar. Das chirurgische Instrument weist eine Instrumentenbasis 2 mit einer Aufnahme 5 auf, in welcher ein Anschlusskörper 3 aufgenommen ist. Die Aufnahme 5 führt innerhalb der Instrumentenbasis 2 zu einer Schaftöffnung 22, in welcher ein Aufnahmeelement mit einer daran angeordneten Drehradanordnung 21 angeordnet ist. Weiterhin ist eine Handhabungsvorrichtung 20 mit einem Daumenring vorgesehen.

Fig. 2 zeigt eine Instrumentenbasis 2 in einer isometrischen Darstellung. Es ist erkennbar, dass die Aufnahme 5 im wesentlichen zylinderförmigen ausgebildet ist, und eine Öffnung entlang einer Anschlussachse H ausbildet. Die Anschlussachse H verläuft insbesondere auf einer Ebene, welche durch einen Längsschnitt durch das chirurgische Instrument, wie in Fig. 1 dargestellt, aufgespannt ist. Des Weiteren kann die Anschlussachse H in einem beliebigen Winkel auf dieser Ebene verlaufen.

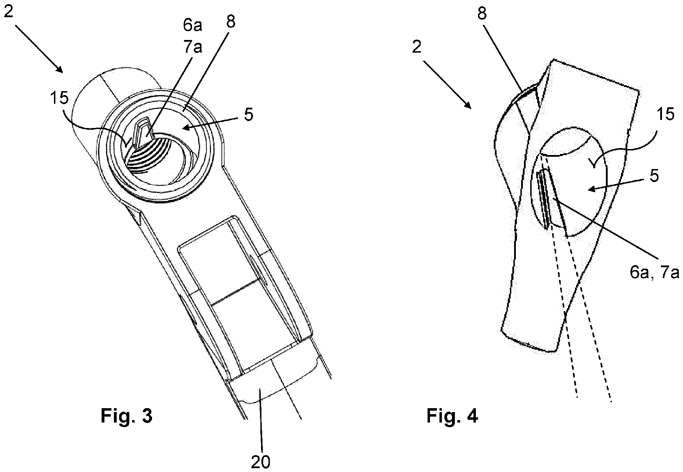

Fig. 3 zeigt eine Aufnahme 5 in einer Instrumentenbasis 2. In der Aufnahme 5 ist ein Ausrichtungsabschnitt 6a vorgesehen, der als Vorsprung 7a ausgebildet ist. Der Ausrichtungsabschnitt 6a ist an einer Innenoberfläche 15 der Aufnahme 5 ausgeformt. Der Ausrichtungsabschnitt 6a bildet daher eine Art Anschlag aus, gegen welchen sich der Anschlusskörper 3 (nicht dargestellt) abstützen kann. In der dargestellten Ausführungsform ist der Ausrichtungsabschnitt 6a beanstandet zu einer Einführöffnung 8 angeordnet. Des Weiteren verläuft der Ausrichtungsabschnitt 6a entlang der Anschlussachse H. In einer weiteren nicht dargestellten Ausführungsform kann der Ausrichtungsabschnitt 6a eine abweichende Geometrie aufweisen, und beispielsweise an die Einführöffnung 8 angrenzen. Des Weiteren können mehrere derartige Ausrichtungsabschnitte 6a an der Innenoberfläche 15 vorgesehen sein.

Fig. 4 zeigt einen Querschnitt durch die Aufnahme aus Fig. 3. Der Querschnitt verläuft durch die Instrumentenbasis 2 und durch den Ausrichtungsabschnitt 6a. Es ist erkennbar, dass die beiden Seitenkanten des Ausrichtungsabschnitts 6a, bzw. des Vorsprungs 7a, konisch aufeinander zu laufen. Bevorzugt sind die Seitenkanten konisch in Richtung der Einführöffnung 8 ausgeformt, wie in Fig. 4 dargestellt. Die dargestellte Verjüngung des Vorsprungs 7a in Richtung der Einführöffnung 8 ist in mehrerlei Hinsicht vorteilhaft. Wird das Bauteil beispielsweise durch ein Spritzgussverfahren hergestellt, muss ein Kern verwendet werden, der aus dem fertigen Spritzteil beim Entformen herausgezogen werden muss. Parallel gegenüberliegende Flächen erzeugen hierbei unnötig Reibung und sind deshalb konstruktiv zu vermeiden. Die konischen Flanken können daher vorteilhaft Entformungsschrägen ausbilden. Weiterhin resultiert ein Vorteil bei der Montage, da sich die beiden Teile, d. h. der Anschlusskörper 3 und die Aufnahme 5 mit den daran angeordneten Ausrichtungsabschnitten 6a, 6b, beim Zusammenfügen sehr leicht "finden". Wird der Anschlusskörper 3 weiter eingeschoben, kann der zunehmend breiter werdende Vorsprung 7a den Anschlusskörper 3 auf eine endgültige Position ausrichten, in welcher der Anschlusskörper 3 spielfrei in der Aufnahme 5 verbleibt.

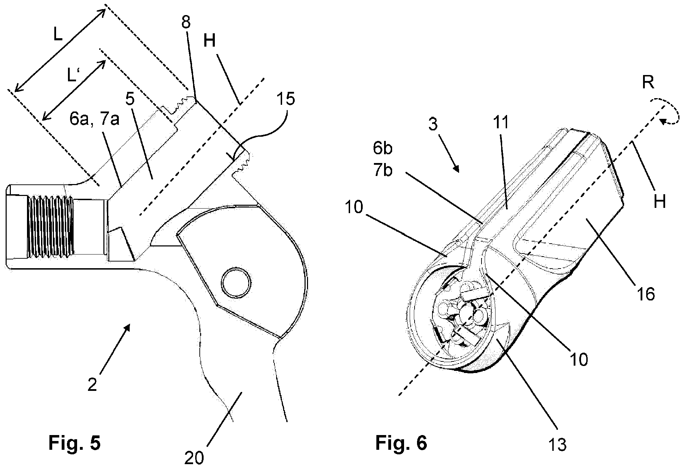

Fig. 5 zeigt einen Längsschnitt durch die Aufnahme aus Fig 3. Es ist erkennbar, dass die Länge L` des Ausrichtungsabschnitts 6a kürzer als die Länge L der kompletten Aufnahme 5 ausgeformt ist. In der dargestellten Ausführungsform verläuft der Ausrichtungsabschnitt 6a, ausgebildet als ein Vorsprung 7a, über zumindest die Hälfte der Länge L der Aufnahme 5. Es ist ebenso denkbar, dass der Ausrichtungsabschnitt 6a an einer anderen Position an der Innenoberfläche 15 angeordnet ist.

Fig. 6 zeigt einen Anschlusskörper 3. Der Anschlusskörper 3 kann wie dargestellt in die Aufnahme 5 gemäß Fig. 5 eingeführt werden. Dazu weist der Anschlusskörper 3 einen Ausrichtungsabschnitt 6b auf, der als Rücksprung 7b ausgebildet ist. Der Rücksprung 7b ist ebenso entlang der Anschlussachse H ausgerichtet. Des Weiteren verläuft in dem Rücksprung 7b eine Kontaktfläche 11 eines ersten Pols 12. Der Rücksprung 7b bildet daher eine Aufnahme für das Kontaktblech 11 aus, wobei das Kontaktblech 11 eine geringere Dicke als eine Tiefe des Rücksprungs 7b aufweist. Dadurch kann der Ausrichtungsabschnitt 6b in einer bereits vorhandenen Geometrie an dem Anschlusskörper 3 ausgeformt werden. Des Weiteren ist eine Einführkontur 10 an dem Anschlusskörper 3 vorgesehen, welche stetig in den Ausrichtungsabschnitt 6b übergeht. Die Einführkontur 10 dient zum Einführen des Ausrichtungsabschnitts 6b wenn der Anschlusskörper 3 in einem Winkel ungleich des vorbestimmten Drehwinkels R auf der Anschlussachse H ausgerichtet ist. Die Einfuhrkontur 10 ist daher als schräge bzw. gekrümmte Fläche ausgebildet, die eine zylindrische Oberfläche 13 an dem Anschlusskörper 3 schneidet. Durch eine derartige Geometrie kann das Einführen des Anschlusskörpers 3 in die Aufnahme 5 vereinfacht werden.

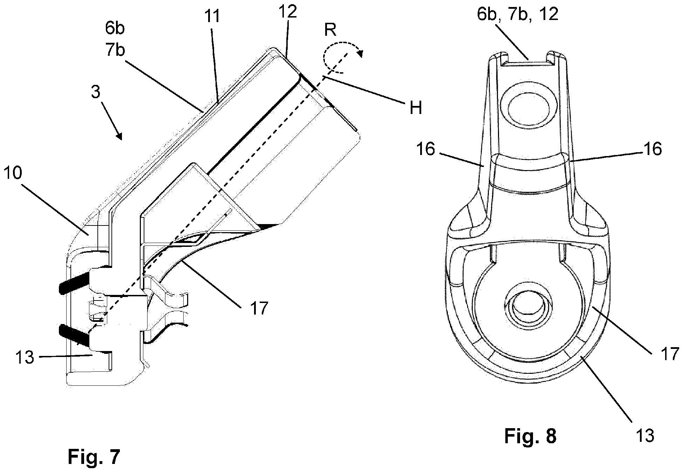

Fig. 7 zeigt einen Längsschnitt durch den Anschlusskörper 3 aus Fig. 6. Es ist erkennbar, dass das Kontaktblech 11 über die Länge des Anschlusskörpers 3 verläuft, und an einem Ende einen ersten Pol 12 ausbildet, der an dem gegenüberliegenden Ende mit Kontaktlaschen ausgeformt ist. Die Kontaktlaschen werden durch die zylindrische Oberfläche 13 und die Einführkontur 10 geschützt. Des Weiteren ist ein Rücksprung 17 an dem Anschlusskörper 3 vorgesehen, durch welchen Material eingespart werden kann, und ein Querschnitt ausgeformt wird, der durch ein Spritzgussverfahren einfach hergestellt und einfach entformt werden kann.

Fig. 8 zeigt eine weitere Ansicht eines Anschlusskörpers 3. Es ist erkennbar, dass der Anschlusskörper 3 konisch zuläuft, wobei an einem Endbereich 2 Ausnehmungen 16 vorgesehen sind, und an dem gegenüberliegenden Endbereich die zylindrische Oberfläche 13 angeordnet ist. Die Ausnehmungen 16 können einen Teil der Verjüngung ausbilden. Des Weiteren können die Ausnehmungen 16 als Griff und/oder Kennzeichnung zur rotatorischen Ausrichtung in dem vorbestimmten Drehwinkel R dienen. Dadurch kann allein optisch erkannt werden, an welcher Position der Ausrichtungsabschnitt 6b mit dem ersten Pol 12 ausgerichtet sein muss, um symmetrisch in die Aufnahme 5 eingeführt zu werden.

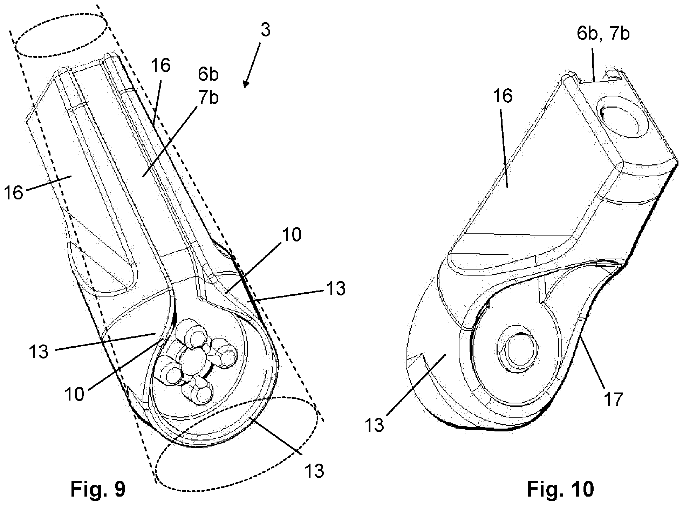

Fig. 9 zeigt eine weitere Ansicht eines Anschlusskörpers 3, wobei die konische Form durch gestrichelte Linien gekennzeichnet ist. Eine derartige Form eignet sich insbesondere für ein Spritzgussverfahren, da diese lediglich runde Flächen aufweist, welche vorteilhaft entformt werden können.

Fig. 10 zeigt eine weitere Ansicht eines Anschlusskörpers 3. In dieser Ansicht sind die Rücksprünge 17 an der Rückseite des Anschlusskörpers 3 erkennbar, wodurch der Anschlusskörper 3 materialsparend ausgeführt werden kann.

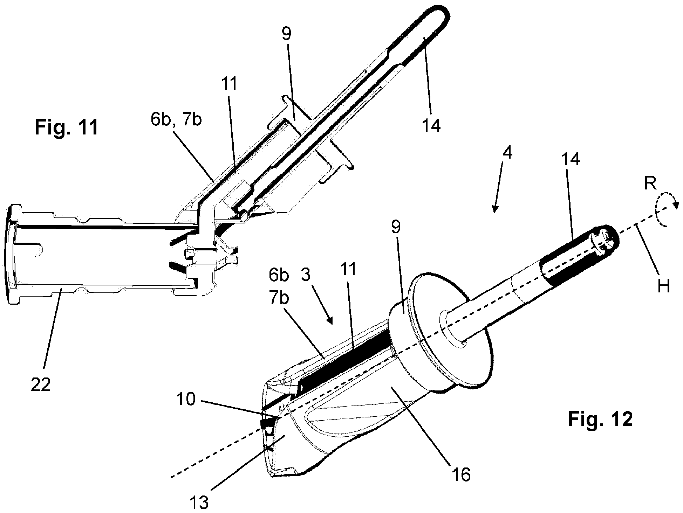

Fig. 11 zeigt eine weitere Ansicht eines Anschlusskörpers 3 mit Steckverbinder 9. Innerhalb des Steckverbinders 9 ist ein zweiter Pol 14 vorgesehen, der ebenso zu dem gegenüberliegenden Ende am Anschlusskörper 3 geführt ist. An dem gegenüberliegenden Ende kann der Anschlusskörper 3 mit einem Aufnahmeelement in der Schaftöffnung 22, gezeigt in Fig. 1, verbunden werden.

Wie in Fig. 12 gezeigt, kann der Steckverbinder 9 an der Seite mit dem Anschlusskörper 3 verbunden werden, an welchen die Ausnehmungen 16 vorgesehen sind. Die Darstellung zeigt eine Art Steckerelement, das als Hoch-Frequenz-Anschluss für einen bipolaren Instrumenten-Handgriff ausgebildet sein kann. Der Anschlusskörper 3 kann dabei eine Art Grundkörper ausbilden, und ist insbesondere aus einem isolierenden Kunststoff ausgebildet. Einer der beiden Pole, insbesondere der erste Pol 12, wird über das Kontaktblech 11 vom Steckverbinder 9 zum Instrumentenschaft übertragen und dort durch vier Kontaktlaschen kontaktiert. Der zweite Pol 14 verläuft im Innern des Steckerelements.

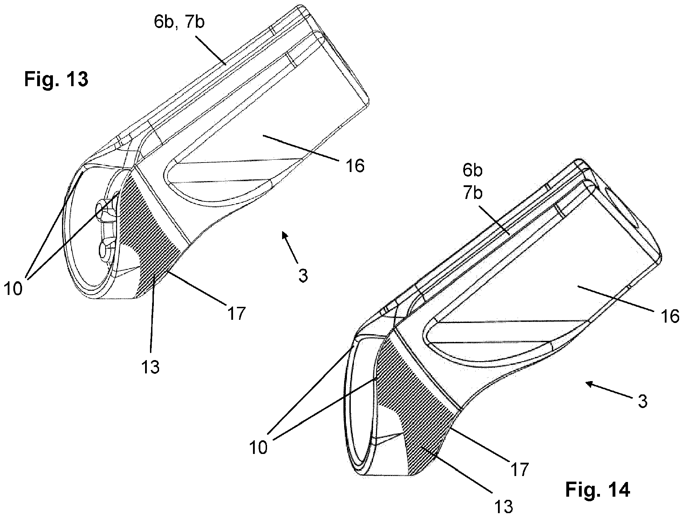

Fig. 13 und Fig. 14 zeigen weitere Ansichten eines Anschlusskörpers 3, wobei die zylindrische Oberfläche 13 als gestrichelte Fläche gekennzeichnet ist. Über die dargestellte gestrichelte Oberfläche können ein passgenaues Ausrichten und ein vereinfachtes Einführen des Anschlusskörpers 3 in die Aufnahme 5 erfolgen. Ein Verhaken beim Einführen kann dabei ausgeschlossen werden. Es ist weiteren erkennbar, dass die Einführkontur 10 die zylindrische Oberfläche 13 schneidet, und dabei quasi einen Teil der zylindrischen Oberfläche entfernt. Dieser Teil dient zum vereinfachten Ausrichten der beiden Ausrichtungsabschnitte 6a und 6b gegeneinander.

Obwohl die vorliegende Erfindung anhand bevorzugter Ausführungsbeispiele vorstehend vollständig beschrieben wurde, ist sie darauf nicht beschränkt, sondern auf vielfältige Art und Weise modifizierbar. So kann beispielsweise die Geometrie des Anschlusskörpers 3 beliebig anders ausgeformt sein, solange die beschriebene Funktion der Ausrichtung durch die Ausrichtungsabschnitt 6a, 6b gewährleistet ist.

### Bezugszeichenliste

- 1: Anschlussanordnung
- 2: Instrumentenbasis
- 3: Anschlusskörper
- 4: Hoch-Frequenz-Anschluss
- 5: Aufnahme
- 6a: Ausrichtungsabschnitt
- 6b: Ausrichtungsabschnitt
- 7a: Vorsprung
- 7b: Rücksprung
- 8: Einführöffnung
- 9: Steckverbinder
- 10: Einführkontur
- 11: Kontaktblech
- 12: erster Pol
- 13: zylindrische Oberfläche
- 14: zweiter Pol
- 15: Innenoberfläche
- 16: Ausnehmung
- 17: Rücksprung
- 20: Handhabungsvorrichtung
- 21: Drehradanordnung
- 22: Schaftöffnung

- H: Anschlussachse
- L, L': Länge
- R: Drehwinkel

## Patentansprüche

1. Anschlussanordnung (1) für ein chirurgisches Instrument oder in einem chirurgischen Instrument, mit:
einer Instrumentenbasis (2), die zur Ansteuerung und/oder Bedienung eines Hoch-Frequenz-Werkzeugs des chirurgischen Instruments ausgebildet ist,
einem Anschlusskörper (3) eines Hoch-Frequenz-Anschlusses (4), wobei der Anschlusskörper (3) zur vorbestimmten Ausrichtung in einer Anschlussachse (H) des Hoch-Frequenz-Anschlusses (4) innerhalb der Instrumentenbasis (2) ausgebildet ist,
wobei die Instrumentenbasis (2) eine Aufnahme (5) für den Anschlusskörper (3) ausbildet und die Aufnahme (5) einen Ausrichtungsabschnitt (6a) aufweist, wobei der Anschlusskörper (3) einen korrespondierenden Ausrichtungsabschnitt (6b) aufweist, und wobei der Anschlusskörper (3) durch Einführen in die Aufnahme (5) durch die Ausrichtungsabschnitte (6a, 6b) in einem vorbestimmten Drehwinkel (R) auf der Anschlussachse (H) selbstausrichtbar ist.

2. Anschlussanordnung (1) nach Anspruch 1,
**dadurch gekennzeichnet,**
**dass** die Ausrichtungsabschnitte (6a, 6b) als ein Vorsprung (7a) und ein dazu korrespondierender Rücksprung (7b) ausgebildet sind.

3. Anschlussanordnung (1) nach Anspruch 2,
**dadurch gekennzeichnet,**
**dass** der Vorsprung (7a) von einer Innenoberfläche (15) der Aufnahme (5) hervorsteht.

4. Anschlussanordnung (1) nach einem der Ansprüche 2 oder 3,
**dadurch gekennzeichnet,**
**dass** der Vorsprung (7a) entlang der Anschlussachse (H) ausgerichtet ist und eine längliche Form aufweist.

5. Anschlussanordnung (1) nach einem der Ansprüche 2 bis 4,
**dadurch gekennzeichnet,**
**dass** der Vorsprung (7a) über zumindest die Hälfte einer Länge (L) der Aufnahme (5) verläuft, wobei die Länge (L) in Richtung der Anschlussachse (H) definiert ist.

6. Anschlussanordnung (1) nach einem der Ansprüche 2 bis 5,
**dadurch gekennzeichnet,**
**dass** der Vorsprung (7a) beabstandet zu einer Einführöffnung (8) der Aufnahme (5) angeordnet ist.

7. Anschlussanordnung (1) nach einem der Ansprüche 2 bis 6,
**dadurch gekennzeichnet,**
**dass** der Vorsprung (7a) zur Ausrichtung des Anschlusskörpers (3) in der Aufnahme (5) zu der Einführöffnung (8) konisch zulaufende Seitenkanten aufweist.

8. Anschlussanordnung (1) nach einem der vorstehenden Ansprüche,
**dadurch gekennzeichnet,**
**dass** der Anschlusskörper (3) konisch ausgebildet ist und sich in Richtung eines Steckverbinders (9) verjüngt.

9. Anschlussanordnung (1) nach einem der vorstehenden Ansprüche,
**dadurch gekennzeichnet,**
**dass** der Anschlusskörper (3) zumindest eine schräge oder gekrümmte Einführkontur (10) aufweist, um ein Einführen der Ausrichtungsabschnitte (6b) zu ermöglichen, wenn der Anschlusskörper (3) in einem Winkel ungleich des vorbestimmten Drehwinkels (R) auf der Anschlussachse (H) ausgerichtet ist.

10. Anschlussanordnung (1) nach einem der Ansprüche 2 bis 9,
**dadurch gekennzeichnet,**
**dass** der Rücksprung (7b) eine Aufnahme für ein Kontaktblech (11) eines ersten Pols (12) ausbildet, wobei das Kontaktblech (11) eine geringere Dicke als eine Tiefe des Rücksprungs (7b) aufweist.

11. Anschlussanordnung (1) nach einem der Ansprüche 9 bis 10,
**dadurch gekennzeichnet,**
**dass** in einem an die Einführkontur (10) anschließenden Endbereich der Rücksprung (7b) stetig in die Einführkontur (10) übergeht.

12. Anschlussanordnung (1) nach einem der vorstehenden Ansprüche,
**dadurch gekennzeichnet,**
**dass** der Anschlusskörper (3) Ausnehmungen (16) aufweist, die als Griff und/oder Kennzeichnung zur rotatorischen Ausrichtung in dem vorbestimmten Drehwinkel (R) des Anschlusskörpers (3) relativ zu der Aufnahme (5) dienen.

13. Anschlussanordnung (1) nach einem der vorstehenden Ansprüche,
**dadurch gekennzeichnet,**
**dass** der Anschlusskörper (3) zumindest teilweise zylindrisch ausgebildet ist.

14. Anschlussanordnung (1) nach Anspruch 8 und Anspruch 12 oder 13,
**dadurch gekennzeichnet,**
**dass** die Ausnehmungen (16) einen Teil der Verjüngung ausbilden und an einer gegenüberliegenden Seite der Anschlusskörper (3) eine zylindrische Oberfläche (13) aufweist.

15. Anschlussanordnung (1) nach Anspruch 9 und 13 oder 14,
**dadurch gekennzeichnet,**
**dass** die Einführkontur (10) die zylindrische Oberfläche (13) schneidet.

16. Anschlussanordnung (1) nach einem der vorstehenden Ansprüche,
**dadurch gekennzeichnet,**
**dass** der Anschlusskörper (5) als einachsig entformbares Spritzgussteil ausgebildet ist.

17. Chirurgisches Instrument, umfassend eine Anschlussanordnung (1) nach einem der vorstehenden Ansprüche,
**dadurch gekennzeichnet,**
**dass** das chirurgische Instrument bipolar oder monopolar betrieben ist.
